# EUROPEAN PATENT APPLICATION

(11) **EP 2 399 986 A1**
(43) Date of publication of application: **28.12.2011**
(21) Application number: 10166904.2
(22) Date of filing: 22.06.2010
(51) Int. Cl.: C12M 3/04

(54) **Bioreactor and method for creating and/or conditioning biological tissues**

(71) Applicant: Ludwig-Maximilians-Universität München, 80539 München (DE)
(72) Inventor: Akra, Bassil, 81249, München (DE); König, Fabian Hugo, 80798, München (DE)
(74) Representative: Lucke, Andreas

(57) **Abstract**

Disclosed herein is a bioreactor (12) for creating and/or conditioning biological tissues and in particular artificial heart valves, vascular grafts and/or cardiovascular patches. The bioreactor (12) comprises a bioreactor housing (30, 32) having a first section (42) and a second section (54), said first section (42) being adapted to receive a nutrition solution, and said second section (54) being adapted to receive said biological tissue, and means (38; 56, 58, 66, 76, 84) for generating and directing a flow of said nutrition solution from said first section (42) to said second section (54) along a first flow path such as to flow along or through at least a portion of said biological tissue, and for directing a back flow of said nutrition solution from said second section (54) to said first section (42) along a second flow path. The flow directing means (56, 58, 66, 76, 84) and said second flow path are contained in said bioreactor housing (30, 32).

## Description

### FIELD OF THE INVENTION

The present invention relates to the field of tissue engineering. In particular, the invention relates to a bioreactor for creating and/or conditioning biological tissues, such as artificial heart valves, vascular grafts and cardiovascular patches.

### RELATED PRIOR ART

Worldwide, every year approximately 300.000 heart valves are implanted to patients. Currently, there are three different types of implants available, namely mechanical heart valves, biological heart valves and homo grafts. While mechanical artificial heart valves have good lifetimes, unfortunately, they bear a severe risk of thrombosis, meaning that patients with artificial mechanical heart valves have to use anticoagulation medication throughout their lives. This is highly undesirable, because due to the decreased coagulation, any injury or surgery involves a significant risk or danger for the patient.

While biological heart valves and homografts have good haemodynamic properties, unfortunately their lifetime is rather limited. In addition, there is a risk that the patient's body does not accept the biological homograft implant or that an inflammatoric reaction may occur.

In order to overcome the abovementioned difficulties, it has been attempted to produce artif-cial heart valves via tissue engineering. Tissue engineering utilizes living cells as engineering materials. For example, if an artificial heart valve is to be produced by tissue engineering, an artificial heart valve is first seeded with smooth muscle cells, then with fibroblasts and finally with endothelium cells, corresponding to the structure of human vessels. It has been shown that with this three layer construction, the attachment of the endothelium cells has been improved as compared to a simple construction, but the attachment is unfortunately generally not strong enough to withstand shear forces that will occur during operation (see Fang Ning-tao, Xie Shang-zhe, Construction of tissue-engineered heart valves by using decellularized scaffolds and endothelial progenitor cells, Chin Med. 120, 2007, Vol. 8, pp. 696-702). It has been observed that the formation of the extra cellular matrix can be improved if the artificial heart valve is subjected to mechanical strain or a constant fluid flow (see Ralf Sodian, Thees Lemke, Matthias Loebe, Simon P. Hoerstrup, New Pulsatile Bioreactor for Fabrication of Tissue-Engineered Patches, Tissue Engineering, March 9, 200 1, pp. 401-405), thereby allowing for an increased stability of the cell layers. This effect has been employed in a certain class of bioreactors, in which the implant is subjected to fluid flow and mechanical strain. Under these conditions, the endothelium cells will align according to the flow direction of the fluid flow (see Helmut Gulbins, et al, Development of an artificial vessel lined with human vascular cells, The Journal of Thoracic and Cardiovascular Surgery, September 2004, pp. 372-377).

A bioreactor according to the preamble of claim 1 is known from DE 199 19 625 C2. Further related bioreactors are known from DE 103 49 688 A1, DE 103 22 024 A1 and DE 100 53 014 A1.

While significant progress has been made in the tissue engineering by exposing the tissue to a dynamic environment, there is still a strong desire to improve the stability of the tissue, in particular for tissue engineered heart valves and vessels. Also, the operation of prior art bioreactors tends to be rather cumbersome and involved.

The problem underlying the invention is to provide a bioreactor for creating and/or conditioning biological tissues as well as a corresponding method that allow to circumvent the abovementioned problems. This problem is solved by a bioreactor according to claim 1 as well as a method according to claim 15. Preferable embodiments are defined in the dependent claims.

### SUMMARY OF THE INVENTION

The bioreactor of the invention comprises a bioreactor housing having a first section and a second section. The first section is adapted to receive a nutrition solution, cell suspension and/or medicine and the second section is adapted to receive the biological tissue. For example, the biological tissue could be an artificial graft, patch or heart valve in its production state by tissue engineering, but the invention is not limited to this application. Further, the bioreactor of the invention comprises means for generating and directing a flow, particularly a pulsatile flow, of the nutrition solution, cell suspension and/or medicine from the first section to the second section along a first flow path such as to flow along or through (e.g. in case of a heart valve) at least a portion of said biological tissue, and for directing a back flow of said nutrition solution, cell suspension and/or medicine from said second section to said first section along a second flow path, wherein the flow direction means and the second flow path are contained in the bioreactor housing.

In the bioreactor of DE 199 19 625 C2, a nutrition solution is also directed along a first flow path from a first section to a second section such as to flow through the biological tissue, which is a heart valve in this prior art. The nutrition solution is then discharged from the second section to the first section via an external tube and an external reservoir.

In contrast to this, according to the invention both, the flow direction means as well as the second flow path are contained within the bioreactor housing. This way, the external tubing can be omitted, which has proven to be advantageous for two reasons. First of all, the sterilization of the external tubes is rather difficult. It is observed that the quality of the tissue created or conditioned in the bioreactor crucially depends on the degree of sterility. It is suspected that any external tubing is a source of imperfect sterility and may thus compromise the result of the tissue engineering. Second, the external tubing makes the bioreactor as a whole more bulky and the assembly thereof - which has to take place under sterile conditions as well ― more cumbersome.

By keeping the second flow path as well as the flow directing means entirely within the bioreactor housing, these problems can be solved.

In a preferred embodiment, the flow directing means comprise a wall portion disposed between said first and second sections, said wall portion comprising a central opening for allowing a flow of said nutrition solution, cell suspension and/or medicine from said first section to said second section along said first flow path, and one or more openings disposed radially outward from said central opening allowing a flow path of said nutrition solution, cell suspension and/or medicine from said second section to said first section along said second flow path. This type of flow directing means allows to concentrate the first flow path in a central portion of the bioreactor, where the biological tissue can best be placed, and to provide a backflow along said second flow path in a radially outward or circumferential portion of the bioreactor.

In a preferred embodiment, the flow directing means comprise at least one element to favor a certain flow direction in the first or second flow path, and in particular a valve means associated with said central opening and/or said one or more radially outward openings. With such means for favoring a certain flow direction, a circular flow along said first and second flow paths can be induced by simply pressurizing the nutrition solution, cell suspension and/or medicine in the first section of the bioreactor housing, as will be further explained with reference to a preferred embodiment.

Preferably, the flow directing means comprise a flow directing element disposed at the end of said first flow path for directing the flow of said nutrition solution, cell suspension and/or medicine from a central portion of said second section to a circumferential portion thereof. In particular, the flow directing element can be disc-shaped and may have a central opening allowing the nutrition solution, cell suspension and/or medicine to pass from a first side to a second side thereof and a guiding surface on its second side for guiding the flow in said radially outward direction. Such flow directing element allows to direct the nutrition solution, cell suspension and/or medicine along the second flow path by simple means and in particular allows to dispense with the exterior tubing known from prior art bioreactors.

In a preferred embodiment, the means for generating said flow of said nutrition solution, cell suspension and/or medicine along said flow path comprise a flexible membrane or diaphragm disposed between said first section and a third section of said bioreactor, wherein said third section is adjacent to said first section on a side opposite to the second section. Herein, the membrane is adapted to receive a pressure and/or a pressurized medium for operating the same. Using such membrane, the nutrition solution, cell suspension and/or medicine in the first section can be pressurized and thus forced to flow along said first flow path while hermetically sealing the first section in order to maintain sterility.

In a preferred embodiment, the bioreactor housing consists of a transparent material, and in particular acrylic glass, glass or plastic. Due to the transparency of the material, the filling and the pH of the nutrition solution can be checked by visual inspection. A further advantage of an acrylic glass, glass or plastic housing is that it can be easily and efficiently sterilized by formaldehyd or ethylene oxide sterilization.

Preferably, the second section of said bioreactor housing is adapted to receive a removable holding frame for holding said biological tissue. The biological tissue can for example be attached to the removable holding frame by sewing while the holding frame is outside the bioreactor housing, and can then be inserted to a corresponding receptacle in the second section. The use of such removable holding frame and a corresponding receptacle in the second section adds to the improved handling of the bioreactor of the invention.

Preferably, the first section of the bioreactor housing comprises at least one port for introducing and/or discharging nutrition solution, cell suspension and/or medicine to and from the bioreactor housing along a flow path different from said first and second flow paths.

In a particularly preferred embodiment, the bioreactor housing comprises a sensor means for sensing parameters and/or the time evolution of parameters indicative of the creation and/or conditioning process, and in particular for sensing one or more of the following parameters: pressure, flow, temperature, CO₂ concentration and pH of the nutrition solution. Based on these parameters, the creation and/or conditioning process can be optimized, and in particular the operation of the flow generating means, such as pulse rate, pressure amplitudes or flow amplitude can be suitably controlled. This allows to generate biological tissues with improved quality and stability.

In a preferred embodiment, the second section comprises a removable housing part, in particular lid portion, allowing access to the content of the second section. This way the aforementioned removable holding frame can be easily placed or removed from the second section.

In a further preferred embodiment, the second section comprises at least one opening allowing the pressure inside the second section to adapt to atmospheric pressure. This allows to generate the flow along the said first and second flow paths without generating an excessive pressure in the second section. Preferably, the opening comprises a sterile filter.

In a preferred embodiment, the second section of the bioreactor comprises at least one optical access portion allowing optical access to the biological tissue inside the bioreactor housing. In the simplest case, the optical access portion can be a suitable window allowing visual inspection of the biological tissue by the naked eye. Preferably, however, the optical access portion is adapted to receive an optical instrument such as an endoscope or CCD camera, thereby allowing a careful and high resolution inspection of the tissue and optionally also a recording and documentation of the images. By closely optically inspecting the progress of the tissue creation or conditioning, the operation of the bioreactor can be suitably adapted such as to obtain a biological tissue of improved quality. Note that the optical access portion allows only optical access, but no physical access to the inside of the bioreactor housing. Accordingly, the sterility inside the bioreactor housing is not compromised. Also, this means that the optical instruments such as the endoscope or CCD camera do not have to be sterilized prior and after inspection, which greatly adds to the convenient operation of the bioreactor of the invention.

In a preferred embodiment, the bioreactor housing comprises separate parts that are connectible with each other via a threaded connection. This way, the separate parts of the bioreactor housing can be simply screwed together such as to assemble the bioreactor housing. This can be done most easily and conveniently and even under sterile conditions. Herein, the separate parts of the bioreactor housing may comprise one of the following: a lower portion resembling the third section of the bioreactor housing, a middle portion resembling said first section and at least a part of said second section of said bioreactor housing and a lid portion.

In a preferred embodiment, the bioreactor can also be provided in a modular fashion, where different numbers of separate parts may be assembled via threaded connections, or where parts can be selected from a set of available parts of different size, such as to adapt the size of the bioreactor to the size of the biological tissue or the corresponding holding frame. In particular, it is advantageous if the lower portion resembling the third section and the middle portion resembling the first and at least a part of the second section are formed by separate parts, since the interior of these parts can be separated by the membrane located inbetween. This means that it is sufficient to sterilize the middle portion and the membrane, while a sterilization of the lower portion is not as crucial since it is separated from the middle portion and thus from the first and second sections containing the nutrition solution, cell suspension and/or medicine and the biological tissue by the membrane. Again, this adds to the improved handling and operation of the bioreactor of the invention.

According to prior art, typically separate external devices such as respiration pumps or the like are used to operate the flow generating means. In contrast, according to a preferred embodiment of the invention, a system for creating and/or conditioning biological tissues is provided that comprises a bioreactor according to one of the above embodiments and driving means for operating said means for flow generation, wherein the bioreactor and the driving means are integrated with each other in a common assembly or mounted on a common platform. This way, a compact and self sustained system can be provided that can be placed in conventional incubator devices. Since the driving means are integrated with the bioreactor or at least mounted on a common platform, the system only needs to be connected with an electrical power supply for the driving means, but not for example with any external pressure supply or the like.

The system may also comprise heating means and heating control means integrated with the bioreactor in a common assembly or mounted on a common platform. This way, the system is entirely self-sustained and does not even have to be moved to and from an incubator. In particular, the temperature control means may be adapted to control the temperature based on one or more of the parameters sensed by the aforementioned sensing means, thus allowing to optimize the temperature in the biological tissue creation and/or conditioning process.

In a preferred embodiment, the driving means comprise an airtight cylinder connected to the third section, a mechanically moved piston for generating a variation of pressure inside said cylinder and a drive system for moving said piston. Such driving means are robust and reliable under operation and at the same time cheaper than for example a respiration pump.

Preferably, the system comprises a control unit for controlling the driving means, wherein the control unit is adapted to control the generation of flow of said nutrition solution, cell suspension and/or medicine according to a program for creating and/or conditioning biological tissues and in particular in response to one or more parameters sensed by the aforementioned sensing means.

### BRIEF DESCRIPTION OF THE FIGURES

Fig. 1 is a perspective view of a system for creating and/or conditioning biological tissues,
Fig. 2 is a sectional view of the bioreactor employed in the system of Fig. 1,
Fig. 3 is a perspective view of a holding frame for holding a biological tissue,
Fig. 4 is an exploded view of the holding frame of Fig. 3, and
Fig. 5 is a perspective view of a flow directing element employed in the bioreactor of Fig. 2.

### DESCRIPTION OF THE PREFERRED EMBODIMENT

For the purposes of promoting an understanding of the principles of the invention, reference will now be made to the preferred embodiment illustrated in the drawings and specific language will be used to describe the same. It will nevertheless be understood that no limitation of the scope of the invention is thereby intended, such alterations and further modifications in the illustrated device and method and such further applications of the principles of the invention as illustrated therein being contemplated therein as would normally occur now or in the future to one skilled in the art to which the invention relates.

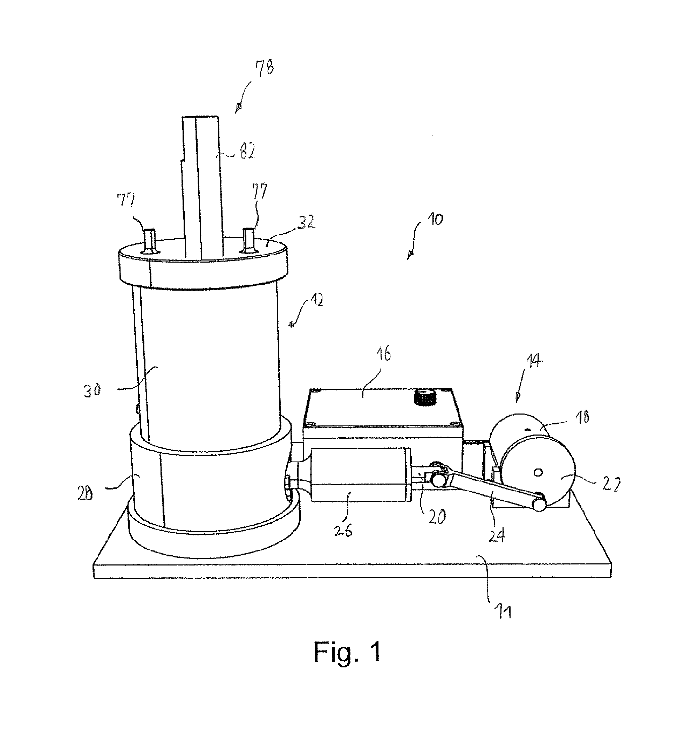

Fig. 1 shows a system 10 for creating and/or conditioning biological tissues. The system 10 comprises a mounting platform 11 an which a bioreactor 12, a driving means 14 and a control unit 16 are mounted. The driving means 14 comprise an electric motor 18 for operating a piston 20 via an eccentric disk 22 and a connecting rod 24. The piston 20 is disposed in an airtight cylinder 26 so that only its end portion connected with the connecting rod 24 can be seen in Fig. 1.

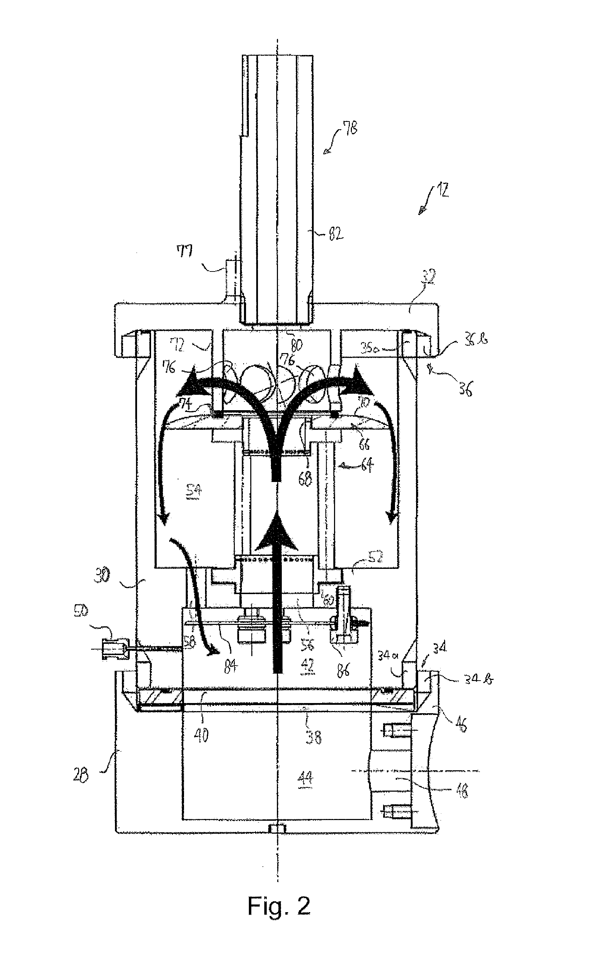

The structure of the bioreactor 12 will next be described with reference to Fig. 2 in which the bioreactor 12 is shown in a sectional view.

As is shown in Fig. 2, the bioreactor 12 comprises a housing comprising a lower portion 28, a middle portion 30 and a lid portion 32, In the preferred embodiment shown in Fig. 2, each of the lower portion 28, middle portion 30 and lid portion 32 are made from acrylic glass, glass or plastic. The lower portion 28 and the middle portion 30 can be easily assembled via a threaded connection 34 comprising a male thread 34a disposed on the lower end of the middle portion 30 and a female thread 34b disposed on the upper end of the lower portion 28. Likewise, the middle portion 30 and the lid portion 32 are connected by a threaded connection 36 comprised of a male thread 36a disposed on the upper end of the middle portion 30 and a female thread 36b disposed on the lid portion 32.

Between the lower portion 28 and the middle portion 30, a flexible membrane or diaphragm 38 held by a ring shaped membrane holder 40 is disposed. When the lower and middle portions 28, 30 are tightened together via the threaded connection 34, the membrane holder 40 and the membrane 38 held thereby are fixed in position, thereby separating a first section 42 within the middle portion 30 and a third section 44 in the lower portion 28 from each other. Further shown in Fig. 2 is a mounting means 46 for mounting airtight cylinder 26 such that its interior is in fluid communication with the third section 44 via an opening 48. Accordingly, by operating the piston 20 in the airtight cylinder 26, the pressure in the cylinder 26 and accordingly the pressure in the third section 44 can be controlled. By varying the pressure in the third section 44, the flexible membrane 38 is operated for generating a flow of nutrition solution, cell suspension and/or medicine in the middle portion 30 of the bioreactor 12 in a manner described in more detail below. Consequently, the membrane 38 forms a means for generating a flow of nutrition solution, cell suspension and/or medicine, which is operated by the driving means 14 comprised of the electric motor 18, eccentric disk 22, connecting rod 24, piston 20 and airtight cylinder 26.

In the middle portion 30 of the bioreactor 12, a port 50 is provided for introducing nutrition solution, cell suspension and/or medicine to and/or discharging the same from the first section 42. The middle portion 30 further comprises a horizontal wall portion 52 disposed between the first section 42 and a second section 54. The wall portion 52 comprises a central opening 56 and a number of openings 58 disposed radially outward from the central opening 56. While in the sectional view of Fig. 2 only one radially outward opening 58 is shown, it is to be understood that a plurality of such radially outward openings 58 are disposed circumferentially around said central opening 56.

An annular valve disk 84 is disposed in the first section 42 and slidingly held by a number of vertical screws 86. When the valve disk plate 84 is in the lower position shown in Fig. 2, it allows a flow of nutrition solution, cell suspension and/or medicine from the second section 54 to the first section 42 via the radially outward openings 58.

Conversely, when the valve disk plate 84 is lifted to its upper position abutting on the wall portion 52, it covers each of the radially outward openings 52, thereby preventing a flow of nutrition solution, cell suspension and/or medicine from the first section 42 to the second section 54.

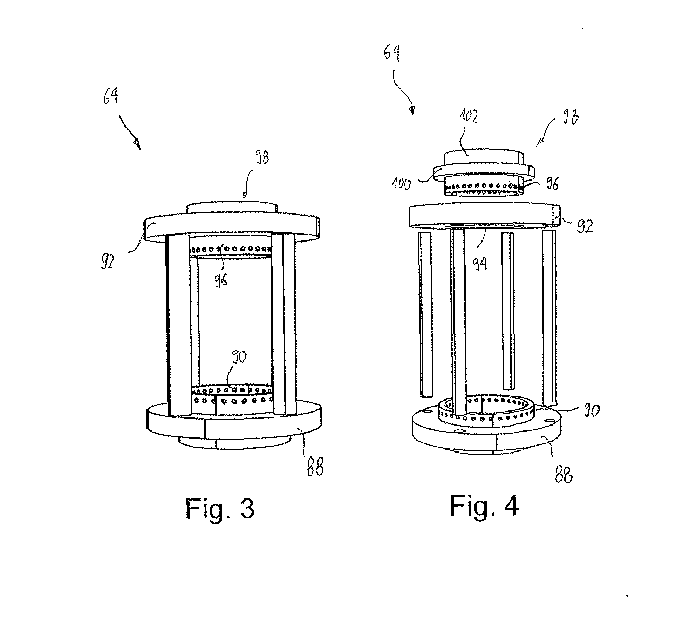

On the upper side of wall portion 52, a recess 60 is formed that is adapted to receive a removable holding frame 64. The holding frame 64 serves to hold the biological tissue that is to be created and/or conditioned in the bioreactor 12. The holding frame 64 will be described in more detail below with reference to the perspective views of Figs. 3 and 4.

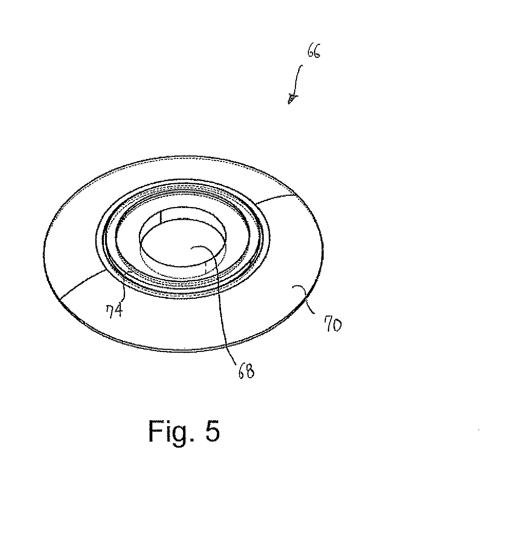

In the vicinity of the upper end of the holding frame 64, a disc shaped flow directing element 66 is disposed. The flow directing element 66 has a central opening 68 allowing a flow of nutrition solution, cell suspension and/or medicine to pass from its lower to its upper side. On the upper side of the flow directing element 66, a flow directing or guiding surface 70 is formed which is adapted to guide a flow of nutrition solution, cell suspension and/or medicine in a radially outward direction. The flow directing element 66 is also shown in a perspective view in Fig. 5.

As can be seen in Fig. 2, in the preferred embodiment the flow directing element 66 rests on top of the holding frame 64, wherein the central opening 68 of the flow directing element 66 receives an end portion of the holding frame 64.

As is further shown in Fig. 2, a cylindrical portion 72 extends downward from the lid portion 32 such as to engage with an annular groove 74 formed on the upper side of the flow directing element 66 (see Fig. 5). Consequently, when the lid portion 32 is screwed onto the middle portion 30 via the threaded connection 36, the holding frame 64 and the flow directing element 66 are firmly held in place between the cylindrical portion 72 and the wall portion 52. In the wall of the cylindrical portion 72, a number of openings 76 are formed for allowing a flow of nutrition solution, cell suspension and/or medicine to pass through.

As can be seen in Fig. 1 and 2, two openings 77 are provided in the lid portion 32 allowing the pressure inside the second section 54 to adapt to the atmospheric pressure. Each of the openings 77 is provided with a sterile filter that allows gas exchange, particularly within an incubator. At least one of the openings 77 may be used for introducing and/or discharging said nutrition solution, cell suspension and/or medicine to/from said bioreactor 12, particularly to/from said second section 54.

On the upper side of the lid portion 32, an optical access portion 78 is formed. The optical access portion 78 comprises a window portion 80 and a receiving portion 82 for receiving an optical instrument, such as an endoscope or CCD camera. When an endoscope or CCD camera is inserted into the receiving portion 82, it can be used to record optical images of the biological tissue (not shown in Fig. 2) disposed in the holding frame 64. Consequently, the process of creation and/or conditioning of the biological tissue can be monitored by optical inspection at any time. Also, the creation and/or conditioning process can be automatically recorded employing recording means associated with the endoscope or CCD camera. By allowing to monitor the process of the biological tissue creation/conditioning at high resolution and in a simple manner, the tissue engineering can be suitably controlled and optimized. While the optical instrument such as an endoscope or CCD camera can be brought very closely to the biological tissue held in the holding frame 64, it does not need to enter the second section 54 and thus does not come in contact with the surrounding of the biological tissue, so that the optical instrument does not need to be sterilized. This greatly facilitates the optical inspection and monitoring of the tissue engineering process.

With reference to Figs. 3 and 4, the holding frame 64 is described in more detail. Fig. 3 shows the holding frame 64 in an assembled state and Fig. 4 and exploded view thereof. The holding frame 64 comprises a lower part 88 having a flange portion 90. The flange portion 90 has a plurality of holes 92 for allowing to attach the biological tissue or the precursor thereof using surgical stitches.

The holding frame 64 further comprises an upper portion 92 having a central opening 94 for receiving a flange portion 96 of an upper member 98. In the assembled state, the upper member 98 rests on top of the upper portion 92 with the flange portion 96 extending through the central opening 94 in the upper portion 92. By choosing among different upper members with flange portions 96 of different lengths, the effective length of the holding frame 64, i.e. the distance between the flange portions 90 and 96 of the lower portion and the upper member can be freely chosen. This way, a holding frame 64 of a universal design can be easily adapted to the desired size suitable for the biological tissue to be created or conditioned. In a preferred embodiment, all members of the holding frame 64 are made from Teflon.

As can be further seen from Fig. 4, the upper member 98 comprises a larger diameter section 100 and a smaller diameter section 102. In the assembled state, the lower annular surface of the larger diameter section 100 rests on the top surface of upper portion 92. Further, with reference to Figs. 2 and 5, in the assembled state, the flow directing element 66 rests on the upper annular surface of the larger diameter section 100, where the smaller diameter section 102 is inserted into the central opening 68 of the flow directing element 66.

Next, the function and operation of the system 10 will be described.

In the preferred embodiment, the system 10 is used for a rather soft and gentle preconditioning of the biological tissue, in particular of an artificial heart valve. That is to say, in the preferred embodiment the biological tissue processed will already have been seeded or populated with cells in a different bioreactor. For this purpose, the tissue will already be fixed in the holding frame 64 which is compatible with the bioreactor in which the seeding took place. After the preconditioning processing in the system 10 of the present invention, the biological tissue will then be removed from the bioreactor 12 and transferred to a different bioreactor which is designed for the conditioning under increased stress that is similar to the mechanical stresses occurring after the tissue, such as an artificial heart valve, is implanted. While in the preferred embodiment the system 10 of the invention is therefore used for such preconditioning, i.e. for a step between the seeding of the biological tissue and the final conditioning, it is by no means limited to it. Instead, the system 10 may also be used for seeding of a biological tissue or for final conditioning.

In the system 10 as shown in Fig. 1, the lower portion 28 of the bioreactor is permanently fixed to the mounting platform 11. Prior to use, the lower portion 28 is sterilized, for example by formaldehyd or ethylene oxide sterilization while mounted on the mounting platform 11. All the remaining separate components of the bioreactor 12 are also sterilized by formaldehyd or ethylene oxide sterilization prior to assembly.

Next, the membrane 38 fixed in the corresponding membrane holder 40 is placed in a corresponding recess at the upper edge of lower portion 28, and the middle portion 30 is screwed to the lower portion 28 using the threaded connection 34, thereby establishing a hermetic seal between the lower and middle portions 28, 30 and fixing the membrane holder 40 and membrane 38 in position. Please note that ordinary sealing means can be used between the portions of the bioreactor 12, which, however, will not be described herein.

Next, the holding frame 64 with the biological tissue attached thereto will be inserted in the corresponding recess 60 in the wall portion 52 as shown in Fig. 2. As mentioned before, typically the holding frame will be taken from a different bioreactor in which the seeding of the biological tissue with cells has already taken place.

Next, the flow diverting element 66 is placed on top of the upper portion 92 of the holding frame 64 such that its lower side rests on the upper side of the larger diameter section 100 of the upper member 98, such that the smaller diameter portion 102 of the upper member 98 at least partially extends through the central opening 68 of flow directing element 66.

Finally, the lid portion 32 is screwed to the middle portion 30 via the threaded connection 36 in an airtight manner. Upon screwing the lid portion 32 to the middle portion 30, the lower edge of the cylindrical portion 72 extending downward from the lid portion 32 engages with the annular groove 74 on the upper side of the flow directing element 66, thereby holding the holding frame 64 and the flow directing element 66 in position.

Note that all of the separate components of the bioreactor, i.e. middle portion 30, holding frame 64, flow directing element 66 and lid portion 32 can be easily sterilized, for example by formaldehyd or ethylene oxide sterilization. Also, note that all of these components can be easily and quickly assembled under sterile conditions.

After assembly of the bioreactor 12, a nutrition solution, cell suspension and/or medicine is introduced to the first section 42 via port 50. Then, the control unit 16 controls the electric motor 18 such as to drive the piston 20 via the eccentric disk 22 and the connecting rod 24, thereby raising the pressure in the airtight cylinder 26 as well as in the third section 44 connected therewith. In response to an increase in pressure in the third section 44, the flexible membrane 38 warps in an upward direction and pressurizes the nutrition solution, cell suspension and/or medicine in the first section 42. This in turn causes the valve disk plate 84 to rise such as to block the radially outward openings 58 provided in the wall portion 52. Accordingly, the pressurized nutrition solution, cell suspension and/or medicine can only escape from the first section 42 via the central opening 56 in the wall portion 52 such as to flow from the first section 42 along a first flow path through (in case of an artificial heart valve) or along the biological tissue held in the holding frame 64 and through openings 76 provided in the cylindrical portion 72. In Fig. 2, the first flow path is represented by the thick arrows.

The nutrition solution, cell suspension and/or medicine flowing through the openings 76 in the cylindrical portion 72 is then guided in a radially outward direction by the flow directing or guiding surface 70 of the flow directing element 66 such as to flow back to the first section 42 along a second flow path which is a radially outward flow path extending along the circumference of the second section 54 and through the radially outward openings 58, once the valve disk plate 84 has moved downwards to the position shown in Fig. 2 upon pressure drop in the first section 42. The second flow path is resembled by the thin arrows in Fig. 2.

Accordingly, by simply operating the membrane 38 and using the central opening 56, the opening 76 in the cylindrical portion 72, the flow directing element 66, the radially outward openings 58 and the valve disk plate 84 as passive flow directing means, it is possible to generate and direct a flow of the nutrition solution, cell suspension and/or medicine from the first section 42 along a first flow path such as to flow along or through at least a portion of the biological tissue, and back from the second section 54 to the first section 42 along a second flow path different from said first flow path. In particular, all of the flow directing means as well as the second flow path are contained in the bioreactor housing. This is different from prior art, where the second flow path would be formed by external tubes connecting first and second sections of the bioreactor. This, however, leads to difficulties with sterilizing the tubes and also makes the bioreactor more clumsy and inconvenient to handle.

As has been mentioned in the introductory part, the stability of the extra cellular matrix of the biological tissue will depend on the way the process of conditioning or preconditioning is carried out. Accordingly, it is important to very delicately control the flow of the nutrition solution, cell suspension and/or medicine, i.e. the frequency and amplitude of flow pulses. The latter can be precisely controlled by the control unit 16. In the preferred embodiment, the control unit 16 will control the operation of the membrane 3 8 and thus the flow of nutrition solution, cell suspension and/or medicine depending on one or more parameters and/or the time evolution of parameters indicative of the creation and/or conditioning process, which may be sensed with appropriate sensing means disposed in the bioreactor 12 (not shown). In particular, the control unit 16 may control the operation of the membrane 38 based on one or more of the following parameters: pressure, flow, temperature, CO₂ concentration and pH of the nutrition solution.

In addition, it is advantageous to constantly monitor the processing of the biological tissue such as to adapt the conditioning to the current condition thereof. According to the preferred embodiment, an endoscope or CCD camera (not shown) is inserted to the receiving portion 82 of the optical access portion 78, such as to monitor the biological tissue through the window portion 80.

Since the bioreactor 12 and the driving means 14 are mounted to a common mounting platform 11, the whole system 10 can be easily transferred to a standard incubator. In particular, while most prior art bioreactors 12 are operated by external pressure generating means, such as respirator pumps, the driving means 14 of the system 10 of the invention allows to generate the operating pressure in a self-sustained way, in the sense that it only needs to be connected to an ordinary electrical power source. Accordingly, the system can be easily operated when placed in a standard incubator.

While the system 10 as shown in Fig. 1 can be placed as a whole in an ordinary incubator, in an alternative embodiment the system 10 may further comprise heating means, gas exchange (not shown), heat control and CO₂ sensor means allowing to control the temperature and the CO₂ concentration within the bioreactor housing such as to form an entirely autarkic system for creating and/or conditioning of biological tissues.

The embodiments described above and the accompanying figures merely serve to illustrate the method according to the present invention, and should not be taken to indicate any limitation of the method. The scope of the patent is solely determined by the following claims.

### LIST OF REFERENCE SIGNS

10 System for creating and/or conditioning of biological tissues
11 mounting platform
12 bioreactor
14 driving means
16 control unit
18 8 electric motor
20 piston
22 eccentric disk
24 connecting rod
26 airtight cylinder
28 lower portion
30 middle portion
32 lid portion
34 threaded connection
36 threaded connection
38 flexible membrane
40 membrane holder
42 first section
44 third section
46 mounting means for airtight cylinder 26
48 opening
50 port for nutrition solution, cell suspension and/or medicine
52 wall portion
54 second section
56 central opening
58 radially outward opening
60 recess
64 holding frame
66 flow directing element
68 central opening of flow directing element 66
70 flow directing surface
72 cylindrical portion
74 annular groove
76 opening in cylindrical portion 72
77 opening in lid portion 32
78 optical access portion
80 window portion
82 receiving portion
84 valve disk plate
86 screw
88 lower portion of holding frame 64
90 flange portion
92 upper portion
94 central opening in upper portion 92
96 flange portion
98 upper member
100 larger diameter section
102 smaller diameter section

## Claims

1. A bioreactor (12) for creating and/or conditioning biological tissues and in particular artificial heart valves, vascular grafts and cardiovascular patches, comprising:
a bioreactor housing (30, 32) having a first section (42) and a second section (54), said first section (42) being adapted to receive a nutrition solution, cell suspension and/or
medicine, and said second section (54) being adapted to receive said biological tissue, and
means (38; 56, 58, 66, 76, 84) for generating and directing a flow of said nutrition solution, cell suspension and/or medicine from said first section (42) to said second section (54) along a first flow path such as to flow along or through at least a portion of said biological tissue, and for directing a back flow of said nutrition solution, cell suspension and/or medicine from said second section (54) to said first section (42) along a second flow path, **characterized in that**
said flow directing means (56, 58,66, 76, 84) and said second flow path are contained in said bioreactor housing (30, 32).

2. The bioreactor (12) according to claim 1, wherein said flow direction means comprise a wall portion (52) disposed between said first and second sections (42, 54), said wall portion (52) comprising a central opening (56) for allowing a flow of said nutrition solution, cell suspension and/or medicine from said first section (42) to said second section (54) along said first flow path,
and one or more openings (58) disposed radially outward from said central portion (56) allowing a flow of said nutrition solution, cell suspension and/or medicine from said second section (54) to said first section (42) along said second flow path.

3. The bioreactor (12) according to one of the preceding claims, wherein said flow directing means comprise at least one element (84) to favour a certain flow direction in the first or second flow path, and in particular, a valve means associated with said central opening (56) and/or said one or more radially outward openings (58).

4. The bioreactor according to one of the preceding claims, wherein said flow directing means comprises a flow directing element (66) disposed at the end of said first flow path for directing the flow of said nutrition solution, cell suspension and/or medicine from a central portion of said second section (54) to a circumferential portion thereof, and in particular a disc shaped flow directing element (66) having
- a central opening (68) for allowing said nutrition solution, cell suspension and/or medicine to pass from a first side to a second side thereof, and
- a guiding surface (70) on its second side for guiding the flow in said radially outward direction.

5. The bioreactor (12) according to one of the preceding claims, wherein said means for generating said flow of said nutrition solution, cell suspension and/or medicine along said flow paths comprise a flexible membrane (38) disposed between said first section (42) and a third section (44) of said bioreactor (12), said third section (44) being adjacent to said first section (42) on a side opposite to said second section (54), wherein said membrane (38) is adapted to receive a pressure and/or a pressurized medium for operating the same.

6. The bioreactor (12) according to one of the preceding claims, wherein said bioreactor housing (30, 32) consists of transparent material, and in particular, acrylic glass, glass or plastic.

7. The bioreactor according to one of the preceding claims, wherein said second section (54) of said bioreactor housing is adapted to receive a removable holding frame (64) for holding said biological tissue, and/or
the second section (54) comprises a removable housing part, in particular a lid portion (32), said removable housing part (32) allowing access to the content of said second section (54), and/or
wherein said second section (54) comprises at least one opening (77) allowing the pressure inside said second section (54) to adapt to the atmospheric pressure, wherein said opening (77) preferably comprises a sterile filter allowing gas exchange, particularly within an incubator, and/or
wherein said second section (54) comprises at least one opening (77) for introducing and/or discharging said nutrition solution, cell suspension and/or medicine to/from said bioreactor (12), particularly to/from said second section (54).

8. The bioreactor (12) according to one of the preceding claims, wherein said first section (42) of said bioreactor housing comprises at least one port (50) for introducing and/or discharging said nutrition solution, cell suspension and/or medicine to and from said bioreactor housing along a flow path different from said first or second flow paths.

9. The bioreactor (12) according to one of the preceding claims, wherein said bioreactor housing comprises sensor means for sensing parameters and/or time evolution of parameters indicative of said creation and/or conditioning process, and in particular for sensing one or more of the following parameters: pressure, flow, temperature, CO₂ concentration and pH of the nutrition solution.

10. The bioreactor (12) according to one of the preceding claims, wherein said second section (54) comprises at least one optical access portion (78) allowing optical access to said biological tissue inside said bioreactor housing, wherein said optical access portion (78) is preferably adapted to receive an optical instrument such as an endoscope or CCD camera.

11. The bioreactor (12) according to one of the preceding claims, wherein the bioreactor housing comprises separate parts that are connectable with each other via a threaded connection (34, 36), wherein said separate parts may comprise one or more of the following:
a lower portion (28) resembling said third section (44) of said bioreactor housing, a middle portion (30) resembling said first section (42) and at least a part of said second section (54) of said bioreactor housing, and a lid portion (32).

12. A system (10) for creating and/or conditioning biological tissues, said system comprising a bioreactor (12) according to one of claims 1 to 11 and driving means (14) for operating said means (38) for flow generation, said bioreactor (12) and said driving means (14) being integrated with each other in a common assembly or mounted on a common platform (11).

13. The system (10) according to claim 11, wherein said driving means (14) comprise an airtight cylinder (26) connected to said third section (44), a mechanically moved piston (20) for generating a variation of pressure inside said cylinder (26), and a drive system (18, 22, 24) for moving said piston (20).

14. The system (10) of claim 12 or 13, further comprising
a control unit (16) for controlling said driving means (14),
said control unit (16) being adapted to control said generation of flow of said nutrition solution, cell suspension and/or medicine according to a program for creating and/or conditioning biological tissue, and in particular in response to one or more parameters sensed by the sensor means of claim 9.

15. A method of creating and/or conditioning biological tissues, in particular artificial heart valves, vascular grafts and cardiovascular patches comprising the following steps:
introducing a nutrition solution, cell suspension and/or medicine into a first section (42) of a bioreactor housing and disposing the biological tissue in a second section (54) of said bioreactor housing,
generating and directing a flow of said nutrition solution, cell suspension and/or
medicine from said first section (42) to said second section (54) along a first flow path such as to flow along or through at least a portion of said biological tissue and
directing a back flow of said nutrition solution, cell suspension and/or medicine from said second section (54) to said first section (42) along a second flow path, wherein
said first and second flow paths are contained in said bioreactor housing,
wherein the processing preferably comprises a preconditioning of the biological tissue after the biological tissue has been seeded and before the biological tissue is finally conditioned.
